# EUROPÄISCHE PATENTANMELDUNG

(11) **EP 1 850 133 A1**
(43) Veröffentlichungstag der Anmeldung: **31.10.2007**
(21) Anmeldenummer: 07007502.3
(22) Anmeldetag: 12.04.2007
(51) Int. Cl.: G01N 33/86, C07K 14/755

(54) **Verfahren zur Bestimmung hoher Ristocetin-Kofaktor-Aktivitäten des von-Willebrand-Faktors**

(30) Priorität: 27.04.2006 DE 102006019520
(71) Anmelder: Dade Behring Marburg GmbH, 35041 Marburg (DE)
(72) Erfinder: Patzke, Jürgen, Dr., 35043 Marburg (DE)

(57) **Zusammenfassung**

Die Erfindung liegt auf dem Gebiet der Gerinnungsdiagnostik und betrifft ein Verfahren zur Bestimmung der Ristocetin-Kofaktor-Aktivität des von-Willebrand-Faktors (VWF:RCo-Aktivität) in Proben, die hohe von-Willebrand-Faktor-Konzentrationen enthalten. Der besondere Vorteil des Verfahrens besteht darin, dass Proben, insbesondere VWF-Konzentrate deren von-Willebrand-Faktor-Gehalt bestimmt werden soll, vor dem Vermischen mit den Testreagenzien nicht mehr verdünnt werden müssen, sofern die VWF:RCo-Aktivität im Testansatz 200 % der Norm nicht überschreitet.

## Beschreibung

Die Erfindung liegt auf dem Gebiet der Gerinnungsdiagnostik und betrifft ein Verfahren zur Bestimmung des von-Willebrand-Faktors in Proben, die hohe von-Willebrand-Faktor-Konzentrationen enthalten. Der besondere Vorteil des Verfahrens besteht darin, dass Proben, deren von-Willebrand-Faktor-Gehalt bestimmt werden soll, vor dem Vermischen mit den erforderlichen Testreagenzien nicht mehr verdünnt werden müssen.

Der von-Willebrand-Faktor (abgekürzt: VWF) ist ein großmolekulares, multimeres Glykoprotein, das aufgrund seiner wichtigen Funktionen in der primären Hämostase, das heißt bei der Thrombozytenadhäsion und -aggregation, auch als Adhäsivprotein bezeichnet wird. Der VWF erfüllt im Falle einer Gefäßverletzung drei wesentliche Aufgaben: Er vermittelt die Adhäsion der Thrombozyten an das Subendothel, er vermittelt die Aggregation der Thrombozyten untereinander und begünstigt dadurch die Thrombusbildung, und er bildet mit dem plasmatischen Blutgerinnungsfaktor VIII (F VIII) einen Komplex, der den F VIII vor vorzeitigem Abbau schützt. Durch Thrombin wird F VIII vom F VIII-VWF-Komplex abgespalten und zu F VIIIa aktiviert und erlangt so seine prokoagulatorische Aktivität, die auch häufig als F VIII:C bezeichnet wird.

Die normale Konzentration des von-Willebrand-Faktor-Antigens (VWF:Ag) im Blut umfasst eine große interindividuelle sowie intraindividuelle Spannbreite. Der Referenzbereich für Normalindividuen (Normalbereich) reicht von 40 % bis 240 % der Norm unter Einbeziehung aller Altersgruppen und des 95 % Konfidenzintervalls. Es ist z. B. bekannt, dass bei Personen mit der Blutgruppe 0 die VWF:Ag-Konzentration im Blut um etwa 25 % geringer ist als bei Personen mit anderen Blutgruppen. Mit zunehmendem Alter steigt der VWF:Ag-Spiegel, so dass der VWF:Ag-Spiegel von Personen, die älter als 70 Jahre alt sind, um ca. 15 % höher ist als der VWF:Ag-Spiegel von Zwanzigjährigen. Während der Schwangerschaft steigt der VWF:Ag-Spiegel kontinuierlich an, unter Umständen auf bis zu 1064 %. Erhöhte Konzentrationen des VWF:Ag werden als Risikofaktor oder Risikoindikator für arterielle Gefäßerkrankungen diskutiert, und auch bei venösen Thromboembolien wurde mehrfach von erhöhten VWF:Ag-Konzentrationen berichtet.

Angeborene oder erworbene Verminderungen oder Funktionsdefekte des VWF werden als von-Willebrand-Syndrom bezeichnet und gehen mit einer Blutungsneigung (Hämophilie) einher, die je nach Schweregrad lebensbedrohliche Formen annehmen kann. Es sind verschiedene Varianten des von Willebrand-Syndroms bekannt: Typ I, verursacht durch eine quantitative VWF:Ag-Verminderung; Typ II mit mehreren Subtypen, verursacht durch einen teilweisen Aktivitätsverlust, welcher oft nicht gleich stark mit einer Verminderung der Antigenkonzentration einhergeht; und Typ III, verursacht durch ein komplettes Fehlen des VWF:Ag. Zur Diagnose eines von-Willebrand-Syndroms bzw. zur Differenzierung der unterschiedlichen Varianten werden verschiedene Testverfahren eingesetzt, wie z. B. immunologische Assays, die eine quantitative Bestimmung der VWF:Ag-Konzentration erlauben sowie funktionelle Assays, die die quantitative Bestimmung der Aktivität des VWF:Ag ermöglichen.

Der sogenannte Ristocetin-Kofaktor-Assay dient der Bestimmung der Ristocetin-Kofaktor-Aktivität des VWF. Die Ristocetin-Kofaktor-Aktivität des VWF, die auch als VWF:RCo bezeichnet wird, ist die Aktivität des VWF, die in Gegenwart von Ristocetin die Aggregation von nativen oder stabilisierten Thrombozyten (Plättchen) bewirkt. In vivo müssen Thrombozyten aktiviert werden, bevor sie aggregieren können. In vitro bewirkt der VWF in Gegenwart von Ristocetin die Aggregation von nicht aktivierten Thrombozyten. Ristocetin ist ein therapeutisch nicht verwendbares Antibiotikum, dessen einziges Einsatzgebiet der Ristocetin-Kofaktor-Assay ist.

Der Ristocetin-Kofaktor-Assay zur Bestimmung der Ristocetin-Kofaktor-Aktivität des VWF wird üblicherweise so durchgeführt, dass die zu untersuchende Probe, bevorzugt eine Plasmaprobe, mit Thrombozyten und mit Ristocetin vermischt wird und die Aggregationsreaktion gemessen wird. Die Aggregation kann visuell bewertet werden, wie z. B. in EP 186 476 A2 beschrieben. Diese semiquantitative Methode wird zunehmend durch die halbautomatische, quantitative aggregometrische Messung ersetzt, bei der üblicherweise die Lichttransmission gemessen wird. Durch die Aggregatbildung wird die Lichtdurchlässigkeit der Thrombozytensuspension erhöht, so dass durch die Messung der Lichtdurchlässigkeit z. B. die Geschwindigkeit der Aggregatbildung bestimmt werden kann. Die Aggregationsreaktion verhält sich proportional zur VWF:RCo-Aktivität der Probe: Je höher z. B. die Geschwindigkeit der Aggregationsreaktion desto größer die VWF:RCo-Aktivität in der Probe. Im Laufe der Zeit wurden verschiedene Variationen des Ristocetin-Kofaktor-Assays entwickelt, die eine vollautomatische präzise Bestimmung der VWF:RCo-Aktivität erlauben. Moderne Gerinnungsmessgeräte, wie z. B. der BCS^{®} Gerinnungsanalyzer (Dade Behring Marburg GmbH, Marburg, Deutschland) messen die Veränderung der optischen Dichte des Testansatzes und bestimmen automatisch die VWF:RCo-Aktivität der Probe in % der Norm.

Bei der klassischen Aggregometrie werden die Reaktionsansätze zur Bestimmung der VWF:RCo-Aktivität permanent gerührt, da die Thrombozytenaggregatbildung ansonsten nicht erfolgt. Eine Modifikation dieses klassischen Verfahrens ist in dem Patentdokument EP 458 330 B1 beschrieben, das die Bestimmung der Aggregationsreaktion bei kontrollierter Zentrifugation während der Messwerterfassung betrifft. Eine andere Modifikation ist in dem Patentdokument EP 1 134 584 B1 beschrieben, das eine modifizierte Bestimmung der Aggregationsreaktion durch Rühren bzw. Nichtrühren des Reaktionsansatzes betrifft.

Die quantitative Bestimmung der VWF:RCo-Aktivität einer Probe erfolgt, indem die Aggregationsreaktion der Probe mit einer Referenzkurve verglichen wird. Referenzkurven werden üblicherweise erstellt, indem eine oder mehrere Referenzproben mit bekannter VWF:RCo-Aktivität stufenweise verdünnt werden und die Aktivitäten gemessen werden. Entspricht die Aktivität einer Referenzprobe der durchschnittlichen Aktivität einer normalen Bevölkerung, so wird sie als 100 % der Norm definiert oder auch als 1 IU (Internationale Einheit). Wird z. B. eine 1:1 verdünnte Referenzprobe mit 100 % der Norm gemessen, so repräsentiert die gemessene VWF:RCo-Aktivität 50 % der Norm.

Wie bereits oben beschrieben wurde, wird beim Ristocetin-Kofaktor-Assay die zu untersuchende Probe mit mindestens einem Reagenz, das Ristocetin und stabilisierte Thrombozyten enthält, zu einem Testansatz vermischt. In den derzeitig bekannten Verfahren zur Bestimmung der VWF:RCo-Aktivität in einer Probe werden Testansätze so erzeugt, dass die VWF:RCo-Aktivität im Testansatz üblicherweise bei etwa 10 % der Norm liegt. In den Richtlinien des NCCLS (National Committee for Clinical Laboratory Standards; seit dem 01. Januar 2005 umbenannt in CLSI, Clinical and Laboratory Standards Institute) zur Bestimmung der VWF:RCo-Aktivität setzt sich der 500 µL Testansatz aus 400 µL einer Thrombozytensuspension, 50 µL einer Ristocetin-Lösung und 50 µL einer unverdünnten oder verdünnten Plasmaprobe zusammen. Die VWF:Ag-Endkonzentration bzw. VWF:RCo-Aktivität bezogen auf das Gesamtvolumen des Testansatzes, wie sie durch Zugabe von unverdünntem normalem Plasma zustande kommt, beträgt also 10 % der Norm. Auch in der weiteren Fachliteratur sind die Testansätze so zusammengesetzt, dass die VWF:Ag-Konzentration bzw. die VWF:RCo-Aktivität im Testansatz immer deutlich unter 30 % der Norm beträgt. Üblicherweise werden dazu die zu untersuchenden Plasmaproben zuvor mit einem geeigneten Puffer oder mit VWF:Ag depletiertem Plasma verdünnt. In Tabelle 1 sind die maximalen VWF:Ag-Konzentrationen bzw. VWF:RCo-Aktivitäten in Testansätzen zur Bestimmung der VWF:RCo-Aktivität, aus verschiedenen exemplarischen Fachartikeln dargestellt.

**Tabelle 1**

| **Literatur** | | **VWF:Ag-Konzentration bzw. VWF:RCo-Aktivität im Testansatz** **(% der Norm)** | **Thrombozyten pro µl Testansatz** |
|---|---|---|---|
| 1. | Allain, J. P. et al. (1975) J. Lab. Clin. Med. 85, 318-327. | **25 %** | **200.000** |
| 2. | Rodeghiero, F. & Castaman, G. (1987) Thromb Haemost. 58, 978-981 | **10 %** | **240.000** |
| 3. | NCLLS. Assays of von Willebrand Factor Antigen and Ristocetin Cofactor Activity; Proposed Guideline. Document H51-P. 2001 (ISBN 1-56238-440-6) | **10 %** | **nicht angegeben** |
| 4. | Federici, A. B. et al. (2004) Haematologica 89, 77-85 | **10 %** | **196.078** |

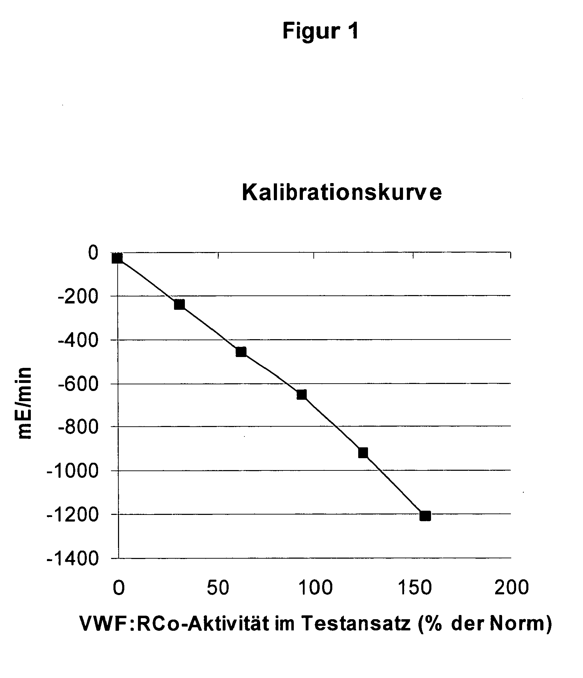

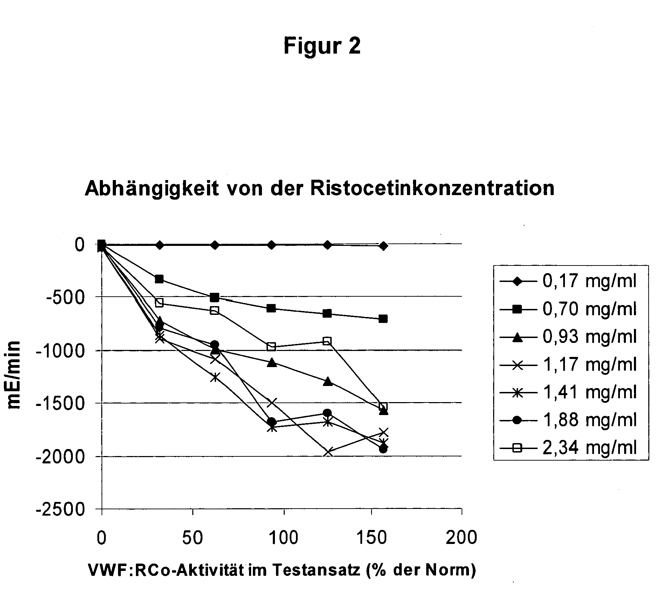

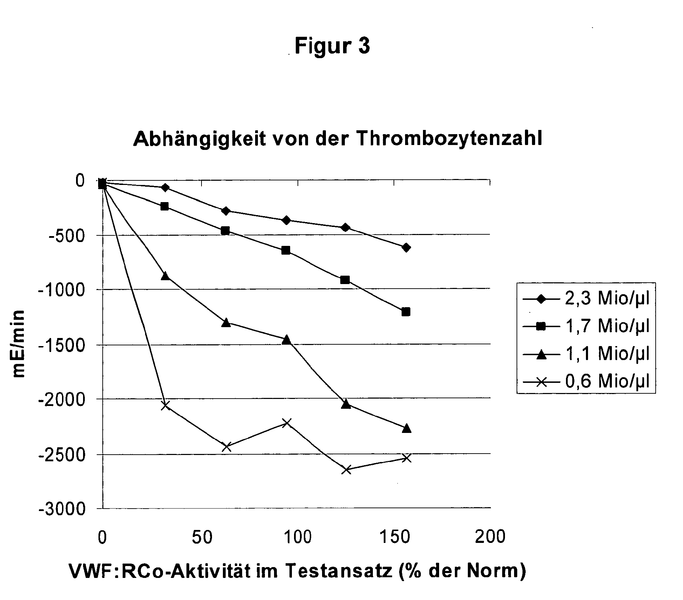

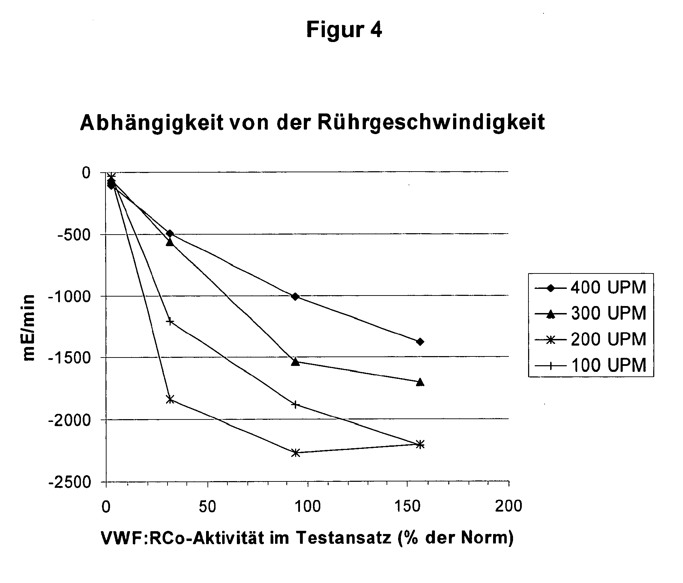

Der Nachteil bei den bisher bekannten Ausführungsformen des Ristocetin-Kofaktor-Assays ist, dass die zu untersuchende Probe üblicherweise vorverdünnt werden muss, wenn die VWF-Aktivität der Probe deutlich höher als die durchschnittliche Aktivität eines Normalplasmas (100 % der Norm) ist. So empfiehlt z. B. das NCCLS in seinen Richtlinien eine stärkere Verdünnung der Probe, wenn die Aggregationsrate größer ist als beim unverdünnt eingesetzten Normalreferenzplasma, weil die Referenzkurve sehr stark abflacht (siehe Figur 1 in NCCLS Document H51-P). Besonders nachteilig wirkt sich die Notwendigkeit zur Verdünnung bei der Bestimmung von hochkonzentrierten VWF-Konzentraten aus, die z. B. für die Substitutionstherapie von von-Willebrand-Syndrom-Patienten vorgesehen sind. Die lyophilisierten VWF-Konzentrate verschiedener Hersteller enthalten typischerweise nach Rekonstitution zwischen 2000 und 10.000 % der Norm VWF. Ein derartiges VWF-Konzentrat ist z. B. ein unter dem Handelsnamen Haemate^{®} P bekanntes Präparat (ZLB Behring GmbH, Deutschland). Zur Bestimmung der VWF:RCo-Aktivität müssen Konzentrate bisher stark verdünnt werden. Verdünnungsschritte sind nicht nur zeitaufwändig, sondern bergen auch immer ein Fehlerrisiko. So kann z. B. allein die Art des Verdünnungsmediums einen erheblichen Einfluß auf die gemessene VWF:RCo-Aktivität haben [Fricke, W. A. et al. (1993) Standards for Assay of von Willebrand Factor Concentrates: A Collaborative Study. Thromb Haemost. 70, 351-356]. Die Möglichkeit einer möglichst geringen Verdünnung wäre zur Minimierung dieser Fehlerquelle besonders wünschenswert.

Auch vor dem Hintergrund, dass in der Praxis VWF:RCo-Assays vorwiegend auf vollautomatischen Analysegeräten durchgeführt werden, wäre es ein erheblicher Fortschritt, wenn es möglich wäre, gegenüber der Norm erhöhte VWF:RCo-Aktivitäten direkt, ohne vorherige Verdünnung der Probe zu messen. Selbst moderne Analysegeräte sind nur eingeschränkt fähig, geringe Volumina zu pipettieren. Das geringste Volumen, das beispielsweise von den Gerinnungsmessgeräten BCS^{®} oder BCT^{®} (Dade Behring Marburg GmbH, Marburg, Deutschland) pipettiert werden kann, liegt bei 3 µL, wodurch es üblicherweise als notwendig angesehen wird, Proben vor Bestimmung der VWF:RCo-Aktivität zu verdünnen.

Der vorliegenden Erfindung lag die Aufgabe zugrunde, ein Verfahren bereit zu stellen, das die Bestimmung der VWF:RCo-Aktivität von Proben ermöglicht, die eine gegenüber der Norm erhöhte VWF:RCo-Aktivität aufweisen. Die Lösung der Aufgabe besteht in der Bereitstellung der in den Ansprüchen beschriebenen erfindungsgemäßen Verfahren.

Gegenstand der vorliegenden Erfindung ist ein Verfahren zur Bestimmung der Ristocetin-Kofaktor-Aktivität des von-Willebrand-Faktors (VWF:RCo-Aktivität) in einer Probe, wobei ein Aliquot der Probe mit Thrombozyten und mit Ristocetin zu einem Testansatz vermischt wird und die Aggregationsreaktion der Thrombozyten gemessen wird. Es wurde gefunden, dass in einem Testansatz hohe VWF:RCo-Aktivitäten präzise gemessen werden können, wenn die VWF:RCo-Aktivität, die im Testansatz enthalten ist, größer ist als 25 % der Norm und 200 % der Norm nicht überschreitet. Bevorzugterweise liegt die VWF:RCo-Aktivität, die im Testansatz enthalten ist, zwischen 30 % und 160 % der Norm.

Der Vorteil des erfindungsgemäßen Verfahrens besteht darin, dass Patientenproben und sogar VWF-Konzentrate nicht mehr verdünnt werden müssen, sofern im Testansatz eine VWF:RCo-Aktivität von 200 % der Norm nicht überschritten wird. Da in vielen Ausführungsformen des Ristocetin-Kofaktor-Assays der Anteil der Probe am Testansatzvolumen etwa 10 % beträgt, können Proben die eine VWF:RCo-Aktivität von bis zu 2.000 % der Norm aufweisen, ohne vorherige Verdünnung zuverlässig gemessen werden. Für Proben, die bekanntermaßen eine höhere VWF:RCo-Aktivität als 2.000 % der Norm aufweisen, wie z. B. VWF- bzw. F VIII:VWF-Konzentrate, kann der Assay entweder so modifiziert werden, dass der Anteil der Probe am Testansatzvolumen kleiner ist als 10 %, oder die Probe wird zuvor verdünnt. Würden z. B. in Beispiel 2 bei gleichbleibendem Gesamtvolumen des Testansatzes (160 µL) anstelle von 5 µL Probe nur 1,6 µL Probe eingesetzt werden, betrüge der Anteil der Probe am Testansatzvolumen 1 %. So könnten Proben mit einer VWF:RCo-Aktivität von bis zu 20.000 % der Norm noch unverdünnt gemessen werden.

Das erfindungsgemäße Verfahren eignet sich zur Bestimmung der VWF:RCo-Aktivität in einer Probe, bei der davon auszugehen ist, dass sie eine VWF:RCo-Aktivität zwischen 600 % und 20.000 % der Norm aufweist. Proben, bei denen davon auszugehen ist, dass sie eine VWF:RCo-Aktivität zwischen 600 % und 20.000 % der Norm aufweisen, sind beispielweise VWF-Konzentrate, für die vom Hersteller eine VWF:RCo-Aktivität im vorgenannten Bereich deklariert wurde. Weitere Typen von Proben, bei denen davon auszugehen ist, dass sie eine VWF:RCo-Aktivität zwischen 600 % und 20.000 % der Norm aufweisen, sind Proben von Blut, Blutplasma oder Blutserum, humaner oder tierischer Herkunft, die von einem Donor stammen, der eine akute oder chronische Entzündung, ein Polytrauma oder eine Sepsis aufweist oder sich in einem postoperativen Zustand befindet oder schwanger ist.

Bevorzugterweise enthält der Testansatz pro Mikroliter etwa 900.000 bis etwa 3.000.000 Thrombozyten, besonders bevorzugt etwa 1.200.000 bis 2.000.000 Thrombozyten. Bei der Thrombozytensuspension, die mit der Probe vermischt wird, kann es sich um native oder bevorzugt um stabilisierte Thrombozyten handeln. Bei den stabilisierten Thrombozytenpräparationen handelt es sich um vorbehandelte Thrombozyten, wie z. B. um Formalin-fixierte Thrombozyten oder Thrombozyten, die mit Proteaseinhibitoren behandelt wurden.

Bevorzugterweise enthält der Testansatz Ristocetin in einer Konzentration von 0,7 bis 2 Milligramm, besonders bevorzugt von 0,9 bis 1,5 Milligramm pro Milliliter Testansatz.

Üblicherweise wird der Testansatz zur Bestimmung der VWF:RCo-Aktivität in einer Probe während der Phase, in der die Messung der Aggregationsreaktion erfolgt, kontinuierlich durchmischt. Die Durchmischung kann z. B. durch Rühren oder Schütteln des Testansatzes erfolgen oder durch Ultraschallbehandlung. Die Durchmischung durch Rühren kann z. B. durch die Verwendung eines Magnetrührers erfolgen, wobei ein im Testansatz befindliches magnetisches Rührstäbchen durch ein angelegtes Magnetfeld in eine rotierende Bewegung versetzt wird. In einer besonders bevorzugten Ausführungsform der Erfindung wird der Testansatz kontinuierlich gerührt bei einer Rührgeschwindigkeit von 100 bis 500 Umdrehungen pro Minute (UPM).

Die folgenden Ausführungsbeispiele dienen der Veranschaulichung des erfindungsgemäßen Verfahrens und sind nicht als Einschränkung zu verstehen.

### Figurenbeschreibung

### Figur 1

Kalibrationskurve für die Bestimmung der VWF:RCo-Aktivität, die gemäß Beispiel 2 mit Kalibratoren erstellt wurde, die VWF:RCo-Aktivitäten von 0 % bis 5000 % der Norm enthielten.

### Figur 2

Kalibrationskurven für die Bestimmung der VWF:RCo-Aktivität mit einer Thrombozytenkonzentration und Rührgeschwindigkeit gemäß Beispiel 2, jedoch mit unterschiedlichen Ristocetinkonzentrationen im Testansatz. Die beste Linearität wurde bei Ristocetinkonzentrationen zwischen 0,7 und 2 mg/mL im Testansatz gemessen.

### Figur 3

Kalibrationskurven für die Bestimmung der VWF:RCo-Aktivität mit einer Ristocetinkonzentration und Rührgeschwindigkeit gemäß Beispiel 2, jedoch mit unterschiedlichen Thrombozytenkonzentrationen im Testansatz. Die beste Linearität wurde bei Thrombozytenkonzentrationen zwischen 0,9 und 3 Millionen (Mio) Thrombozyten pro µL Testansatz gemessen.

### Figur 4

Kalibrationskurven für die Bestimmung der VWF:RCo-Aktivität mit einer Ristocetinkonzentration und einer Thrombozytenkonzentration gemäß Beispiel 2, jedoch mit unterschiedlichen Rührgeschwindigkeiten im Reaktionsansatz während der Messung. Die beste Linearität wurde bei Rührgeschwindigkeiten zwischen 100 und 500 Umdrehungen pro Minute (UPM) gemessen.

### Beispiel 1: Herstellung eines Thrombozyten-Ristocetin-Reagenzes und Herstellung von Kalibratoren definierter VWF:RCo-Aktivität

Zur Herstellung eines Reagenzes zur Verwendung in dem erfindungsgemäßen Verfahren zur Bestimmung der VWF-RCo-Aktivität wurden Formaldehyd-fixierte, humane Thrombozyten in einer phosphatgepufferten Kochsalzlösung suspendiert und mit Ristocetin vermischt, so dass das Reagenz 1,8 Millionen Thrombozyten pro µL und 1,25 mg Ristocetin pro mL enthielt.

Zur Erstellung einer Kalibrationskurve wurde Haemate^{®} P (ZLB Behring GmbH, Deutschland), ein Faktor VIII-VWF-Konzentrat verwendet, das in unverdünntem Zustand eine VWF:RCo-Aktivität von etwa 5.000 % der Norm aufwies. Die VWF:RCo-Aktivität war mit Hilfe der Plättchenagglutinationsmethode unter Verwendung eines kommerziellen Reagenzes (Von Willebrand-Reagenz, Dade Behring Marburg GmbH, Marburg, Deutschland), das stabilisierte Thrombozyten, Ristocetin und EDTA enthielt, bestimmt worden. Das Faktor VIII-VWF-Konzentrat wurde seriell mit isotonischer Kochsalzlösung verdünnt, und es wurden jeweils 50 µL Konzentrat-Verdünnung und 50 µL der Kochsalzlösung (Leerwert) auf eine Glasplatte pipettiert. Zu jedem Tropfen wurden anschließend 50 µL Von Willebrand-Reagenz gemischt und die Agglutination vor einem dunklen Hintergrund visuell überprüft. Der Titer der Probe ist die Verdünnungsstufe, in der eine im Vergleich zum Leerwert deutliche Agglutination zu beobachten ist.

Zur Herstellung von Kalibratoren mit unterschiedlichen VWF:RCo-Aktivitäten wurde Haemate^{®} P mit VWF-Mangelplasma verdünnt. Auf diese Weise wurden Kalibratoren hergestellt, die folgende VWF:RCo-Aktivitäten enthielten:

| | |
|---|---|
| Kalibrator 1 | 5000 % der Norm |
| Kalibrator 2 | 4000 % der Norm |
| Kalibrator 3 | 3030 % der Norm |
| Kalibrator 4 | 2000 % der Norm |
| Kalibrator 5 | 1000 % der Norm |
| Kalibrator 6 | 0 % der Norm. |

Kalibrator 6 enthielt kein Haemate^{®} P und bestand ausschließlich aus VWF-Mangelplasma.

### Beispiel 2: Bestimmung der VWF:RCo-Aktivität

Folgende Schritte wurden automatisch auf dem Gerinnungsmessgerät BCT^{®} (Dade Behring Marburg GmbH, Marburg, Deutschland) durchgeführt:

5 µL der zu untersuchenden Plasmaprobe oder des Kalibrators (siehe Beispiel 1) wurden in ein Reaktionsgefäß gegeben, mit 5 µL VWF-Mangelplasma gemischt und für 15 Sekunden bei 37 °C inkubiert. Anschließend wurden 150 µL des Thrombozyten-Ristocetin-Reagenzes (siehe Beispiel 1) zugegeben. Die Testansätze enthielten eine Ristocetinkonzentration von 1,17 mg/mL und eine Thrombozytenkonzentration von etwa 1,68 Millionen pro µL.

Im Falle der Kalibratoren wurden auf diese Weise folgende VWF:RCo-Aktivitäten im Testansatz erzeugt:

**Tabelle 2**

| | **VWF:RCo-Aktivität des Kalibrators** | **VWF:RCo-Aktivität im Testansatz** |
|---|---|---|
| **Kalibrator 1** | 5000 % der Norm | 156 % der Norm |
| **Kalibrator 2** | 4000 % der Norm | 125 % der Norm |
| **Kalibrator 3** | 3030 % der Norm | 95 % der Norm |
| **Kalibrator 4** | 2000 % der Norm | 62,5 % der Norm |
| **Kalibrator 5** | 1000 % der Norm | 31,3 % der Norm |
| **Kalibrator 6** | 0 % der Norm | 0 % der Norm |

Unter kontinuierlichem Rühren des Testansatzes bei 500 UpM wurde die Extinktion des Testansatzes bei 405 nm über einen Zeitraum von 90 Sekunden gemessen. Anhand der so ermittelten Kinetik der Aggregationsreaktion wurde die Extinktionsänderung (mE/Minute) bestimmt, und es wurde die maximale Extinktionsänderung (Vmax der Extinktionsabnahme) ermittelt.

Für die Erstellung der Kalibrationskurve wurden die Vmax-Werte der verschiedenen Kalibratoren gegen die VWF:RCo-Aktivitäten der Kalibratoren (in % der Norm) aufgetragen. Mittels linearer Verbindung dieser Kalibrationskurvenpunkte wurde die Kalibrationskurve berechnet (siehe auch Figur 1).

Die VWF:RCo-Aktivität einer Plasmaprobe wurde dadurch bestimmt, dass durch den Vergleich des Vmax-Werts der Probe mit der Kalibrationskurve die VWF:RCo-Aktivität der Probe (in % der Norm) bestimmt wurde.

## Patentansprüche

1. Verfahren zur Bestimmung der Ristocetin-Kofaktor-Aktivität des von-Willebrand-Faktors (VWF:RCo-Aktivität) in einer Probe, bei der davon auszugehen ist, dass sie eine VWF:RCo-Aktivität zwischen 600 % und 20.000 % der Norm aufweist, wobei ein Aliquot der Probe mit Thrombozyten und mit Ristocetin zu einem Testansatz vermischt wird und die Aggregationsreaktion der Thrombozyten gemessen wird, **dadurch gekennzeichnet dass**
die VWF:RCo-Aktivität im Testansatz größer ist als 25 % der Norm und 200 % der Norm nicht überschreitet.

2. Verfahren gemäß Anspruch 1 **dadurch gekennzeichnet, dass** die VWF:RCo-Aktivität im Testansatz zwischen 30 und 160 % der Norm beträgt.

3. Verfahren gemäß den Ansprüchen 1 und 2 **dadurch gekennzeichnet, dass** die verwendeten Thrombozyten stabilisiert sind.

4. Verfahren gemäß den Ansprüchen 1 bis 3 **dadurch gekennzeichnet, dass** der Testansatz etwa 900.000 bis etwa 3,0 Millionen, bevorzugterweise 1,2 bis 2,0 Millionen Thrombozyten pro Mikroliter enthält.

5. Verfahren gemäß den Ansprüchen 1 bis 4 **dadurch gekennzeichnet, dass** der Testansatz Ristocetin in einer Konzentration von 0,7 bis 2,0 bevorzugterweise von 0,9 bis 1,5 Milligramm pro Milliliter enthält.

6. Verfahren gemäß den Ansprüchen 1 bis 5 **dadurch gekennzeichnet, dass** der Testansatz kontinuierlich durchmischt wird, bevorzugt durch Rühren bei einer Rührgeschwindigkeit von 100 bis 500 Umdrehungen pro Minute.

7. Verfahren gemäß den Ansprüchen 1 bis 6 **dadurch gekennzeichnet, dass** die Probe, bei der davon auszugehen ist, dass sie eine VWF:RCo-Aktivität zwischen 600 % und 20.000 % der Norm aufweist, unverdünntes humanes Blut, Blutplasma oder Blutserum eines Donors ist, der eine akute oder chronische Entzündung, ein Polytrauma oder eine Sepsis aufweist oder sich in einem postoperativen Zustand befindet oder schwanger ist.

8. Verfahren gemäß den Ansprüchen 1 bis 6 **dadurch gekennzeichnet, dass** die Probe, bei der davon auszugehen ist, dass sie eine VWF:RCo-Aktivität zwischen 600 % und 20.000 % der Norm aufweist, ein von-Willebrand-Faktor Konzentrat ist.
